(19) Europäisches Patentamt
European Patent Office
Office européen des brevets

(11) Publication number: 0 013 470 B1

(12) EUROPEAN PATENT SPECIFICATION

(45) Date of publication of patent specification: 25.05.83

(21) Application number: 79302349.0

(22) Date of filing: 26.10.79

(51) Int. Cl.[3]: C 07 H 17/06, A 61 K 31/70, A 61 K 35/16 //A61K7/16, A61K9/00

# (54) *Rutin poly(H-) sulfates, method for their preparation and pharmaceutical compositions containing them.*

(30) Priority: 04.12.78 US 966423
31.07.79 US 62587

(43) Date of publication of application: 23.07.80 Bulletin 80/15

(45) Publication of the grant of the patent: 25.05.83 Bulletin 83/21

(84) Designated Contracting States: AT BE CH DE FR GB IT NL SE

(56) References cited:
FR - A - 2 043 455
FR - A - 2 067 036

ARZNEIMITTEL FORSCHUNG, vol. 27, 1977, pages 378—379 E. FOLDI-BORCSOK: "Wirkung von Benzopyron auf die durch Rinderserum-Albumin herbeigeführte exsudative Peritonitis bei der Ratte"

The file contains technical information submitted after the application was filed and not included in this specification

(73) Proprietor: AMERICAN CYANAMID COMPANY
Berdan Avenue
Wayne New Jersey 06904 (US)

(72) Inventor: Nair, Vijay Gopalan
1090 Amsterdam Avenue Apt. 15D
New York, N.Y. (US)
Inventor: Bernstein, Seymour
26 Scott Drive
New City, New York (US)

(74) Representative: Allam, Peter Clerk et al,
LLOYD WISE, TREGEAR & CO. Norman House
105-109 Strand
London WC2R 0AE (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 013 470 B1

# Rutin poly(H-)sulfates, method for their preparation and pharmaceutical compositions containing them

The present invention resides in the concept of novel rutin poly(H-)sulfates and salts thereof, useful as inhibitors of the complement system of warm-blooded animals.

Certain sulfated polysaccharides have been reported as having complement inhibiting activity, for example, heparin, *J. Infect. Dis., 44*: 250—253 (1929); carrageenin, *Immunology, B*: 291 (1965); and pentosan polysulfoester, *Chemical Abstracts, 75*: 33179s (1971). However, no art is known which discloses anticomplementary activity for the rutin poly(H-)sulfate salts of the present invention.

A rutin sulfate sodium salt ('rutin water soluble') is commercially available from E. Merck, Darmstadt, West Germany, Catalogue No. 500014. This material, which is useful as an injectable form of Vitamin P, has an analysis for two to four sulfates per molecule (sulfur analysis, S = 5.45%). 'Rutin water soluble' has been tested for complement activity using the tests disclosed herein and found lacking in complement inhibiting activity. In "Arzneimittel Forschung", *27,* 1977, pps 378—378, the antiphlogistic effect of the benzopyrone drug "Venalot" is described. Venalot is a water-soluble rutin (poly(H-)sulfate, sodium salt which contains no more than four sulfate groups per molecule, and like "rutin water soluble" is inactive as a complement inhibitor.

The use of rutin and its derivatives, particularly rutin poly(H-)sulfate, sodium salt as a component of dentifrices is disclosed in French Patent No. 2,067,036. The rutin poly(H-)sulfate salts of the present invention are not, however, disclosed in this reference, nor was it to be expected therefrom that the present rutin salts would possess complement inhibiting activity.

Finally, French Patent No. 2,043,455 describes a process for the preparation of certain mono-, di-, tri- and tetra-propylsulfonic acid salts of rutin, said to be useful in the treatment of circulatory diseases. Again, therefore, the valuable discovery of the present invention is not derivable from this prior art.

The term "complement" refers to a complex group of proteins in body fluids that, working together with antibodies or other factors, play an important role as mediators of immune, allergic, immunochemical and/or immunopathological reactions. The reactions in which complement participates take place in blood serum or in other body fluids, and hence are considered to be humoral reactions.

With regard to human blood, there are at present more than 11 proteins in the complement system. These complement proteins are designated by the letter C and by number: C1, C2, C3 and so on up to C9. The complement protein C1 is actually an assembly of subunits designated C1q, C1r and C1s. The numbers assigned to the complement proteins reflect the sequence in which they become active, with the exception of complement protein C4, which reacts after C1 and before C2. The numerical assignments for the proteins in the complement system were made before the reaction sequence was fully understood. A more detailed discussion of the complement system and its role in the body processes can be found in, for example, Bull. World Health Org., *39,* 935—938 (1968); Ann. Rev. Medicine, *19,* 1—24 (1968); The Johns Hopkins Med. J., *128,* 57—74 (1971); Harvey Lectures, *66,* 75—104 (1972); The New England Journal of Medicine, *287,* 452—454; 489—495; 545—549; 592—596; 642—646 (1972); Scientific American, *229,* (No. 5), 54—66 (1973); Federation Proceedings, *32,* 134—137 (1973); Medical World News, October 11, 1974, pp. 53—66; J. Allergy Clin. Immunol., *53,* 298—302 (1974); Cold Spring Harbor Conf. Cell Proliferation 2/Proteases Biol. Control/229—241 (1975); Ann. Review of Biochemistry, *44,* 697 (1975); Complement in Clinical Medicine, Disease-a-Month, (1975); Complement, *Scope,* December 1975; Annals of Internal Medicine, *84,* 580—593 (1976); "Complement: Mechanisms and Functions", Prentice-Hall, Englewood Cliffs, N. J. (1976); Essays Med. Biochem., *2,* 1—35 (1976); Hospital Practice, *12,* 33—43 (1977); Perturbation of Complement in Disease, Chap. 15 in Biological Amplification Systems in Immunology (Ed. Day and Good), Plenum, New York and London (1977); Am. J. Clin. Pathology, *68,* 647—659 (1977).

The complement system can be considered to consist of three sub-systems: (1) a recognition unit (C1q) which enables it to combine with antibody molecules that have detected a foreign invader; (2) an activation unit (C1r, C1s, C2, C4, C3) which prepares a site on the neighboring membrane; and (3) an attack unit (C5, C6, C7, C8 and C9) which creates a "hole" in the membrane. The membrane attack unit is non-specific; it destroys invaders only because it is generated in their neighborhood. In order to minimize damage to the host's own cells, its activity must be limited in time. This limitation is accomplished partly by the spontaneous decay of activated complement and partly by interference by inhibitors and destructive enzymes. The control of complement, however, is not perfect, and there are times when damage is done to the host's cells. Immunity is, therefore, a double-edged sword.

Activation of the complement system also accelerates blood clotting. This action comes about by way of the complement-mediated release of a clotting factor from platelets. The biologically active complement fragments and complexes can become involved in reactions that damage the host's cells, and these pathogenic reactions can result in the development of immune-complex diseases. For example, in some forms of nephritis, complement damages the basal membrane of the kidney, resulting in the escape of protein from the blood into the urine. The disease disseminated lupus erythematosus belongs in this category; its symptoms include nephritis, visceral lesions and skin eruptions. The

treatment of diphtheria or tetanus with the injection of large amounts of antitoxin sometimes results in serum sickness, an immune-complex disease. Rheumatoid arthritis also involves immune complexes. Like disseminated lupus erythematosus, it is an autoimmune disease in which the disease symptoms are caused by pathological effects of the immune system in the host's tissues. In summary, the complement system has been shown to be involved with inflammation, coagulation, fibrinolysis, antibody-antigen reactions and other metabolic processes.

In the presence of antibody-antigen complexes the complement proteins are involved in a series of reactions which may lead to irreversible membrane damage if they occur in the vicinity of biological membranes. Thus, while complement constitutes a part of the body's defense mechanism against infection it also results in inflammation and tissue damage in the immunopathological process. The nature of certain of the complement proteins, suggestions regarding the mode of complement binding to biological membranes and the manner in which complement effects membrane damage are discussed in Annual Review in Biochemistry, *38*, 389 (1969); Journal of Immunology, *119*, 1—8, 1195, 1358—1364, 1482 (1977).

A variety of substances have been disclosed as inhibiting the complement system, i.e., as complement inhibitors. For example, the compounds 3,3′-ureylenebis[6-(2-amino-8-hydroxy-5-sulfo-1-naphthylazo)benzenesulfonic acid], tetrasodium salt (chlorazol fast pink), heparin and a sulphated dextran have been reported to have an anticomplementary effect, British Journal of Experimental Pathology, *33*, 327—339 (1952). German Patent No. 2,254,893 or South African Patent No. 727,923 discloses certain 1-(diphenylmethyl)-4-(3-phenylallyl)piperazines useful as complement inhibitors. Other chemical compounds having complement inhibiting activity are disclosed in, for example, Journal of Medicinal Chemistry, *12*, 415—419; 902—905; 1049—1052; 1053—1056 (1969); Canadian Journal of Biochemistry, *47*, 547—552 (1969); The Journal of Immunology, *104*, 279—288 (1970); The Journal of Immunology, *106*, 241—245 (1971); The Journal of Immunology, *111*, 1061—1066 (1973); Biochim. Biophys. Acta, *317*, 539—548 (1973); Life Sciences, *13*, 351—362 (1973); Journal of Immunology, *113*, 584 (1974); Immunology, *26*, 819—829 (1974); Journal of Medicinal Chemistry, *17*, 1160—1167 (1974); Biochim. Biophys. Res. Comm., *67*, 225—263 (1975); Ann. N. Y. Acad. Sci., *256*, 441—450 (1975); Journal of Medicinal Chemistry, *19*, 634—639, 1079 (1976); Journal of Immunology, *118*, 466 (1977); Arch. Int. Pharmacodyn., *226*, 281—285 (1977); Biochem. Pharmacol. *26*, 325—329 (1977); Journal Pharm. Sci., *66*, 1367—1377 (1977); Chem. Pharm. Bull., *25*, 1202—1208 (1977); Biochim. Biophys. Acta, *484*, 417—422 (1977) and Journal Clin. Microbiology, *5*, 278—284 (1977).

It has been reported that the known complement inhibitors epsilon-aminocaproic acid and tranexamic acid have been used with success in the treatment of hereditary angioneurotic edema, a disease state resulting from an inherited deficiency or lack of fuction of the serum inhibitor of the activated first component of complement (C1 inhibitor), The New England Journal of Medicine, *286*, 808—812 (1972), *287*, 452—454 (1972); Ann. Intern. Med., *84*, 580—593 (1976); J. Allergy and Clin. Immunology, *60*, 38—40 (1977).

Publications related to the biological use of Suramin compounds for the purpose of inhibiting the complement system, including humans, as determined by the *in vivo* and *in vitro* testing of the blood serum of warm-blooded animals are:

B. Stuber and K. Lang, Arch. Exptl. Path. Pharmacol., *154*, 41—49 (1930) [C. A. *25*, 3067 (1931)]
F. Klopstock, Zeitschrift für Immunitatsforschung und experimentalle Therapie, *75*, 348—354 (1932)
H. J. Schmid, Schweiz. Med. Woch., *96*, 1267—1269 (1966)
K. Lauenstein, Bayer-Symposium I, 25—30 (1969)
J. S. C. Fong and R. A. Good, Clin. Exp. Immunol., *10*, 127—138 (1972)
V. Eisen and C. Loveday, Br. J. Pharmac., *49*, 678—687 (1973)
D. Brackertz and F. Kueppers, Allergol. Et IEt Immunopath., *11*, 163—168 (1974)
E. Raepple, H—U. Hill and M. Loos, Immunochemistry, *13* (3), 251—255 (1976)

It has also been reported that the drug pentosanpolysulfoester has an anticomplementary activity on human serum, both *in vitro* and *in vivo*, as judged by the reduction in total hemolytic complement activity; Pathologie Biologie, *25*, 33—36, *25* (2), 105—108, 25 (3), 179—184 (1977).

It has now been discovered that certain rutin poly(H-)sulfate salts interact with the complement reaction sequence, thereby inhibiting complement activity in body fluids.

This invention is particularly concerned with all pharmaceutically acceptable salts of rutin poly(H-)sulfate having complement inhibiting activity of the general formula:

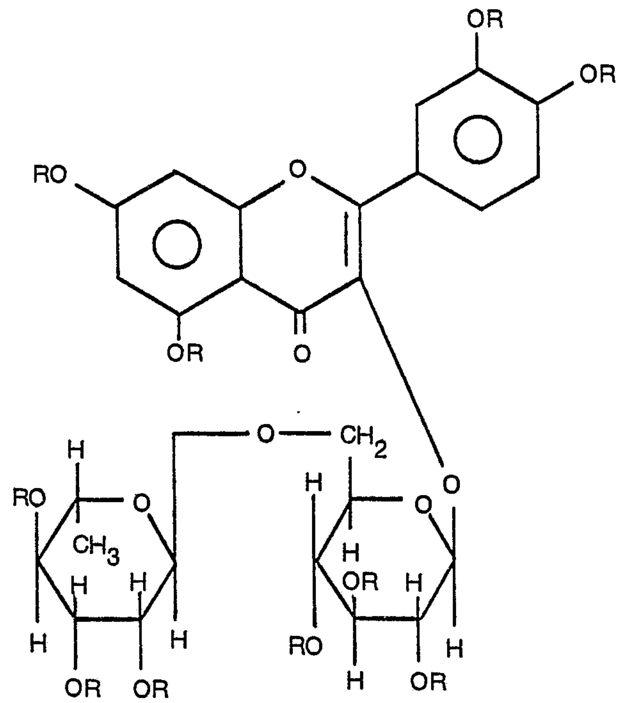

wherein

each R is selected from the group consisting of hydrogen and —$SO_3A$; wherein A is a pharmaceutically acceptable salt cation; with the proviso that at least six of the R groups are —$SO_3A$.

The rutin poly(H-)sulfate salts of the present invention find utility as complement inhibitors in body fluids and as such may be used to ameliorate or prevent those pathological reactions requiring the function of complement and in the therapeutic treatment of warm-blooded animals having immunologic diseases such as rheumatoid arthritis, systemic lupus erythematosus, certain kinds of glomerulonephritis, certain kinds of auto-allergic hemolytic anemia, certain kinds of platelet disorders and certain kinds of vasculitis. Rutin poly(H-)sulfate salts may also be used in the therapeutic treatment of warm-blooded animals having non-immunologic diseases such as paroxysmal nocturnal hemoglobinurea, hereditary angioneurotic edema (such as Suramin Sodium, etc.) and inflammatory states induced by the action of bacterial or lysosomal enzymes on the appropriate complement components as for example, inflammation following coronary occlusion. They may also be useful in the treatment of transplant rejection and as blood culture and transport mediums.

The rutin poly(H-)sulfate salts of this invention may be prepared by the application or adaptation of known methods, for example, as described in *Chemical Reviews, 62:* 549—589 (1962); and U.S. Patent Nos. 3,271,388; 2,923,704; 2,686,779 and 2,697,093; or as described hereinbelow. It will be understood that the present sulfate salts are solvated in varying degrees with water and ethanol. The preferred procedure is described below.

Sulfation Procedure:

A mixture of rutin or rutin water-soluble and trialkylamine-sulfur trioxide complex in dry dimethylformamide is heated at a range from 50°—90°C. for a period of from 20—24 hours. The solution is cooled to room temperature and poured into absolute ethanol with stirring. The solvent is decanted from the separated product which is further purified by leaching with absolute ethanol followed by anhydrous ether. It is then dried *in vacuo.*

Salts of Alkali or Alkaline Earth Metals

The trialkylammonium salt, of rutin poly(H-sulfate) is dissolved in water and is reacted with a 30% aqueous solution of sodium acetate or calcium acetate. The addition of absolute ethyl alcohol is utilized to ensure complete precipitation of the product which is further purified with absolute ethanol. The precipitation step is repeated and purification with absolute ethanol and anhydrous diethyl ether gives the desired product which is desiccated.

DETAILED DESCRIPTION OF THE INVENTION

The following examples describe in detail the preparation and formulation of representative sulfate salts of this invention.

Example 1

Rutin poly(H-)sulfate, trimethylamine salt

A 3.34 g. portion of trimethylamine-sulfur trioxide complex is stirred in warm dimethylformamide to give a clear solution. A 1.22 g. portion of rutin is added and stirring is continued at 65°—70°C. for 20 hours. The solution is cooled to room temperature and poured into absolute ethanol with stirring. The pale yellow gum which separates is collected by decantation, triturated repeatedly with absolute ethanol then ether and dried *in vacuo*, giving the desired product as 3.5 g. of a solvated pale brown gum, (analysis: Found, S = 13.1%). In another preparation, the product analysis for S = 13.5%.

Example 2

Rutin poly(H-)sulfate, sodium salt

A 3.0 g. portion of rutin poly(H-)sulfate, trimethylamine salt is dissolved in water. A 10 ml. portion of 30% aqueous sodium acetate solution is added with swirling and the mixture is allowed to stand at room temperature for 20 minutes. A 100 ml. portion of absolute ethanol is added and the resulting yellow semi-solid is separated by decantation. Trituration with absolute ethanol provides a yellow granular solid which is collected by filtration, washed with absolute ethanol, then ether and dried, giving the desired product as 1.8 g. of a solvated bright yellow powder (analysis: Found, S = 15.3%). In another preparation the product analysis for S = 16.8%.

Example 3

Rutin poly(H-)sulfate, trimethylamine salt prepared from commercially available 'Rutin water-soluble'

A 3.34 g. portion of trimethylamine-sulfur trioxide complex is dissolved in 20 ml. of dimethylformamide by warming the mixture at 65°—70°C. A 1.22 g. portion of 'Rutin water-soluble' (E. Merck, Darmstadt, West Germany) is added and the mixture is stirred at 65°C. for 24 hours. The clear pale yellow solution is cooled to room temperature and 125 ml. of absolute ethanol is added with stirring. The resulting pale brown gum is separated by decantation, triturated with absolute ethanol and dried *in vacuo,* giving 3.1 g. of the desired product as a solvated pale brown gum (analysis: Found, S = 13.9%).

Example 4

Rutin poly(H-)sulfate, sodium salt (original source commercial 'Rutin water soluble')

A 2.9 g. portion of the poly(H-)sulfate, trimethylamine salt prepared in Example 3, is dissolved in 5 ml. of water and 10 ml. of 30% aqueous sodium acetate solution is added. The mixture is filtered, allowed to stand at room temperature for 20 minutes, then 125 ml. of absolute ethanol is added with stirring. The resulting semi-solid is collected and triturated with ethanol. The resulting solid is collected by filtration, washed repeatedly with ethanol, then ether and dried *in vacuo*, giving 2.0 g. of the desired product as a solvated bright yellow powder (analysis: Found, S = 16.5%).

Example 5

Preparation of Compressed Tablet

| Ingredient | mg/Tablet |
|---|---|
| Active Compound | 0.5—500 |
| Dibasic Calcium Phosphate N.F. | qs |
| Starch USP | 40 |
| Modified Starch | 10 |
| Magnesium Stearate USP | 1—5 |

Example 6
Preparation of Compressed Tablet — Sustained Action

| Ingredient | mg/Tablet |
|---|---|
| Active Compound as Aluminum Lake*, Micronized | 0.5—500 (as acid equivalent) |
| Dibasic Calcium Phosphate N.F. | qs |
| Alginic Acid | 20 |
| Starch USP | 35 |
| Magnesium Stearate USP | 1—10 |

*Complement inhibitor plus aluminum sulfate yields aluminum complement inhibitor. Complement inhibitor content in aluminum lake ranges from 5—30%.

Example 7
Preparation of Hard Shell Capsule

| Ingredient | mg/Capsule |
|---|---|
| Active Compound | 0.5—500 |
| Lactose, Spray Dried | qs |
| Magnesium Stearate | 1—10 |

Example 8
Preparation of Oral Liquid (Syrup)

| Ingredient | % W/V |
|---|---|
| Active Compound | 0.05—5 |
| Liquid Sugar | 75.0 |
| Methyl Paraben USP | 0.18 |
| Propyl Paraben USP | 0.02 |
| Flavoring Agent | qs |
| Purified Water qs ad | 100.0 |

Example 9
Preparation of Oral Liquid (Elixir)

| Ingredient | % W/V |
|---|---|
| Active Compound | 0.05—5 |
| Alcohol USP | 12.5 |
| Glycerin USP | 45.0 |
| Syrup USP | 20.0 |
| Flavoring Agent | qs |
| Purified Water qs ad | 100.0 |

Example 10
Preparation of Oral Suspension (Syrup)

| Ingredient | % W/V |
| --- | --- |
| Active Compound as Aluminum Lake, Micronized | 0.05—5 (acid equivalent) |
| Polysorbate 80 USP | 0.1 |
| Magnesium Aluminum Silicate, Colloidal | 0.3 |
| Flavoring Agent | qs |
| Methyl Paraben USP | 0.18 |
| Propyl Paraben USP | 0.02 |
| Liquid Sugar | 75.0 |
| Purified Water qs ad | 100.0 |

Example 11
Preparation of Injectable Solution

| Ingredient | % W/V |
| --- | --- |
| Active Compound | 0.05—5 |
| Benzyl Alcohol N.F. | 0.9 |
| Water for Injection | 100.0 |

Example 12
Preparation of Injectable Oil

| Ingredient | % W/V |
| --- | --- |
| Active Compound | 0.05—5 |
| Benzyl Alcohol | 1.5 |
| Sesame Oil qs ad | 100.0 |

Example 13
Preparation of Intra-Articular Product

| Ingredient | Amount |
| --- | --- |
| Active Compound | 2—20 mg |
| NaCl (physiological saline) | 0.9% |
| Benzyl Alcohol | 0.9% |
| Sodium Carboxymethylcellulose | 1—5% |
| pH adjusted to 5.0—7.5 | |
| Water for Injection qs ad | 100% |

Example 14
Preparation of Injectable Depo Suspension

| Ingredient | % W/V |
|---|---|
| Active Compound | 0.05—5 (acid equivalent) |
| Polysorbate 80 USP | 0.2 |
| Polyethylene Glycol 4000 USP | 3.0 |
| Sodium Chloride USP | 0.8 |
| Benzyl Alcohol N.F. | 0.9 |
| HCl to pH 6—8 | qs |
| Water for Injection qs ad | 100.0 |

Example 15
Preparation of Dental Paste

| Ingredient | % W/W |
|---|---|
| Active Compound | 0.05—5 |
| Zinc Oxide | 15 |
| Polyethylene Glycol 4000 USP | 50 |
| Distilled Water qs | 100 |

Example 16
Preparation of Dental Ointment

| Ingredient | % W/W |
|---|---|
| Active Compound | 0.05—5 |
| Petrolatum, White USP qs | 100 |

Example 17
Preparation of Dental Cream

| Ingredient | % W/W |
|---|---|
| Active Compound | 0.05—5 |
| Mineral Oil | 50 |
| Beeswax | 15 |
| Sorbitan Monostearate | 2 |
| Polyoxyethylene 20 Sorbitan Monostearate | 3 |
| Methyl Paraben USP | 0.18 |
| Propyl Paraben USP | 0.02 |
| Distilled Water qs | 100 |

Example 18
Preparation of Topical Cream

| Ingredient | % W/W |
|---|---|
| Active Compound | 0.05—5 |
| Sodium Lauryl Sulfate | 1 |
| Propylene Glycol | 12 |
| Stearyl Alcohol | 25 |
| Petrolatum, White USP | 25 |
| Methyl Paraben USP | 0.18 |
| Propyl Paraben USP | 0.02 |
| Purified Water qs | 100 |

Example 19
Preparation of Topical Ointment

| Ingredient | % W/W |
|---|---|
| Active Compound | 0.05—5 |
| Cholesterol | 3 |
| Stearyl Alcohol | 3 |
| White Wax | 8 |
| Petrolatum, White USP qs | 100 |

Example 20
Preparation of Spray Lotion (non-Aerosol)

| Ingredient | % W/W |
|---|---|
| Active Compound | 0.05—5 |
| Isopropyl Myristate | 20 |
| Alcohol (Denatured) qs | 100 |

Example 21
Preparation of Buccal Tablet

| Ingredient | g/Tablet |
|---|---|
| Active Ingredient | 0.00325 |
| 6 × Sugar | 0.29060 |
| Acacia | 0.01453 |
| Soluble Starch | 0.01453 |
| F. D. & C. Yellow No. 6 Dye | 0.00049 |
| Magnesium Stearate | 0.00160 |
| | 0.32500 |

The final tablet will weigh about 325 mg. and may be compressed into buccal tablets in flat faced or any other tooling shape convenient for buccal administration.

Example 22
Preparation of Lozenge

| Ingredient | g/Lozenge |
|---|---|
| Active Ingredient | 0.0140 |
| Kompact® Sugar (Sucrest Co.) | 0.7138 |
| 6 x Sugar | 0.4802 |
| Sorbitol (USP Crystalline) | 0.1038 |
| Flavor | 0.0840 |
| Magnesium Stearate | 0.0021 |
| Dye | qs |
| Stearic Acid | 0.0021 |
| | 1.4000 |

The ingredients are compressed into 1.6 cm flat based lozenge tooling. Other shapes may also be utilized.

The rutin poly(H-)sulfate salts of the present invention may be administered internally, e.g., orally, or parenterally, e.g., intra-articularly, to a warm-blooded animal, to inhibit complement in the body fluid of the animal, such inhibition being useful in the amelioration or prevention of those reactions dependent upon the function of complement, such as inflammatory process and cell membrane damage induced by antigen-antibody complexes. A range of doses may be employed depending on the mode of administration, the condition being treated and the particular compound being used. For example, for intravenous or subcutaneous use from about 5 to about 50 mg/kg/day, or every six hours for more rapidly excreted salts, may be used. For intra-articular use for large joints such as the knee, from about 2 to about 20 mg/joint per week may be used with proportionally smaller doses for smaller joints. The dosage range is to be adjusted to provide optimum therapeutic response in the warm-blooded animal being treated. In general, the amount of compound administered can vary over a wide range to provide from about 5 mg/kg to about 100 mg/kg of body weight of animal per day. The usual daily dosage for a 70 kg subject may vary from about 350 mg to about 3.5 g. Unit doses of the acid or salt can contain from about 0.5 mg to about 500 mg.

While in general the sodium salts of the acids of the invention are suitable for parenteral use, other salts may also be prepared, such as those of primary amines, e.g., ethylamine; secondary amines, e.g., diethylamine or diethanolamine; tertiary amines, e.g., pyridine or triethylamine or 2-dimethylaminomethyldibenzofuran; aliphatic diamines, e.g., decamethylenediamine; and aromatic diamines, can be prepared.

It will be appreciated that the sulfatation procedure of the present invention directly provides trialkylamine sulfate salts. Some of these are soluble in water, others are soluble in saline solution, and still others are insoluble and can be used for purposes of preparing suspensions for injection. Furthermore, as well as the sodium salt, those of the alkali metals, such as potassium and lithium; of ammonia; and of the alkaline earth metals, such as calcium or magnesium, may be employed. It will be apparent, therefore, that these salts embrace, in general, derivatives of salt-forming cations.

The rutin poly(H-)sulfate salts of the present invention may also be administered topically in the form of ointments, creams, lotions and the like, suitable for the treatment of complement dependent dermatological disorders.

Moreover, the rutin poly(H-)sulfate salts of the present invention may be administered in the form of dental pastes, ointments, buccal tablets and other compositions suitable for application periodontally for the treatment of periodontitis and related diseases of the oral cavity.

In therapeutic uses, the rutin poly(H-)sulfate salts of this invention may be administered in the form of conventional pharmaceutical compositions. Such compositions may be formulated so as to be suitable for oral or parenteral administration. The active ingredient may be combined in admixture with a pharmaceutically acceptable carrier, which carrier may take a wide variety of forms depending on the form of preparation desired for administration, i.e., oral or parenteral. The sulfate salts can be used in compositions such as tablets. Here, the principal active ingredient is mixed with conventional

tabletting ingredients such as corn starch, lactose, sucrose, sorbitol, talc, stearic acid, magnesium stearate, dicalcium phosphate, gums, or similar materials as non-toxic pharmaceutically acceptable diluents or carriers. The tablets or pills of the novel compositions can be laminated or otherwise compounded to provide a dosage form affording the advantage of prolonged or delayed action or predetermined successive action of the enclosed medication. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids or mixtures of polymeric acids with such materials as shellac, shellac and cetyl alcohol, cellulose acetate and the like. A particularly advantageous enteric coating comprises a styrene maleic acid copolymer together with known materials contributing to the enteric properties of the coating. The tablet or pill may be colored through the use of an appropriate non-toxic dye, so as to provide a pleasing appearance.

The liquid forms in which the novel compositions of the present invention may be incorporated for administration include suitable flavored emulsions with edible oils, such as, cottonseed oil, sesame oil, coconut oil, peanut oil, and the like, as well as elixirs and similar pharmaceutical vehicles. Sterile suspensions or solutions can be prepared for parenteral use. Isotonic preparations containing suitable preservatives are also desirable for injection use.

The term dosage form, as described herein, refers to physically discrete units suitable as unitary dosage for warm-blooded animal subjects, each unit containing a predetermined quantity of active component calculated to produce the desired therapeutic effect in association with the required pharmaceutical diluent, carrier or vehicle. The specification for the novel dosage forms of this invention are indicated by characteristics of the active component and the particular therapeutic effect to be achieved or the limitations inherent in the art of compounding such an active component for therapeutic use in warm-blooded animals as disclosed in this specification. Examples of suitable oral dosage forms in accord with this invention are tablets, capsules, pills, powder packets, granules, wafers, cachets, teaspoonfuls, dropperfuls, ampules, vials, segregated multiples of any of the foregoing and other forms as herein described.

The complement inhibiting activity of the rutin poly(H-)sulfate salts of this invention has been demonstrated by one or more of the following identified tests: (i) Test Code 026 (C1 inhibitor) — This test measures the ability of activated human C1 to destroy fluid phase human C2 in the presence of C4 and appropriate dilutions of the test compound. An active inhibitor protects C2 from C1 and C4; (ii) Test Code 035 (C3—C9 inhibitor) — This test determines the ability of the late components of human complement (C3—V9) to lyse EAC 142 in the presence of appropriate dilutions of the test compound. An active inhibitor protects EAC 142 from lysis by human C3—C9; (iii) Test Code 036 (C-Shunt inhibitor)— In this test human erythrocytes rendered fragile are lysed in autologous serum *via* the shunt pathway activated by cobra venom factor in the presence of appropriate dilutions of the test compound. Inhibition of the shunt pathway results in failure of lysis; (iv) Forssman Vasculitis Test — Here, the well known complement dependent lesion, Forssman vasculitis, is produced in guinea pigs by intradermal injection of rabbit anti-Forssman antiserum. The lesion is measured in terms of diameter, edema and hemorrhage and the extent to which a combined index of these is inhibited by prior intraperitoneal injection of the test compound at 200 mg/kg is then reported, unless otherwise stated; (v) Forssman Shock Test — Lethal shock is produced in guinea pigs by an i.v. injection of anti-Forssman antiserum and the harmonic mean death time of treated guinea pigs is compared with that of simultaneous controls; (vi) Complement Level Reduction Test — In this test, the above dosed guinea pigs, or others, are bled for serum and the complement level is determined in undiluted serum by the capillary tube method of U.S. Patent No. 3,876,376 and compared to undosed control guinea pigs; and (vii) Cap 50 Test — Here, appropriate amounts of the test compound are added to a pool of guinea pig serum *in vitro,* after which the undiluted serum capillary tube assay referred to above is run. The concentration of compound inhibiting 50% is reported, (viii) Guinea Pig Intraperitoneal Test (GPIP) — Guinea Pigs weighing about 300 g. are dosed intraperitoneally (i.p.) with 200 mg./kg. of the test compound dissolved in saline and adjusted to pH 7—8. Approximately 0.4 ml. blood samples, taken by orbital sinus puncture 30 minutes and 1 hour after injections, are collected directly into centrifuge tubes; 5 ml. blood samples, taken by decapitation 2 hours after injection are collected directly into diSPo® beakers. The samples were allowed to clot, centrifuged, and the resultant sera were assayed for complement activity using the capillary complement assay. Percent inhibition are calculated by comparison with simultaneous controls. The results appear in Table I together with results of Test Code 026, 035, 036, Cap 50, % inhibition and Forssman Shock. Table I shows that the preferred sodium sulfate salt of the invention possesses highly significant *in vitro* and *in vivo* complement inhibiting activity in warm-blooded animals.

TABLE I

Biological Activities

| Compound | *In vitro* activity | | | | *In vivo* activity (Guinea Pig) Intraperitoneal % Inhibition Time (minutes) | | | Forssman Shock (minutes) |
|---|---|---|---|---|---|---|---|---|
| | 026* | 035* | 036* | Cap 50* | 30 | 60 | 120 | |
| Rutin | N | N | N | | | | | |
| Rutin Water-soluble (E. Merck) (Analysis S = 5.45%) | N | N | N | | | | | |
| Rutin poly(H-)sulfate trimethylamine salt (Analysis S = 13.1%) | +2** | N | N | > 500 | | | | |
| Rutin poly(H-)sulfate sodium salt (Analysis S = 15.3%) | +7 | N | N | 69 | –79 | –89 | –89 | 17.2 (Control 3.3) |

* Code designation for tests employed as referred herein.

** Activity in wells, a serial dilution assay. Higher well number indicates higher activity. The serial dilutions are two-fold.

N = Inactive.

**Claims for the Contracting States: BE CH DE FR GB IT NL SE**

1. A rutin poly(H-)sulfate of the formula:

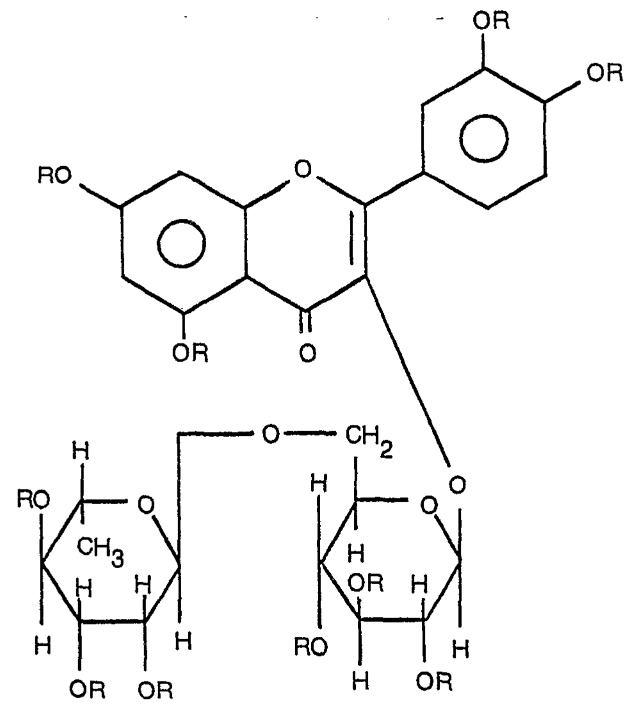

wherein

each R is selected from the group consisting of hydrogen and —$SO_3A$; wherein A is a pharmaceutically acceptable salt cation; with the proviso that at least six of the R groups are —$SO_3A$.

2. The rutin poly(H-)sulfate according to Claim 1, rutin poly(H-)sulfate, trimethylamine salt.

3. The rutin poly(H-)sulfate according to Claim 1, rutin poly(H-)sulfate, sodium salt.

4. A method for the preparation of a rutin poly(H-)sulfate of the formula:

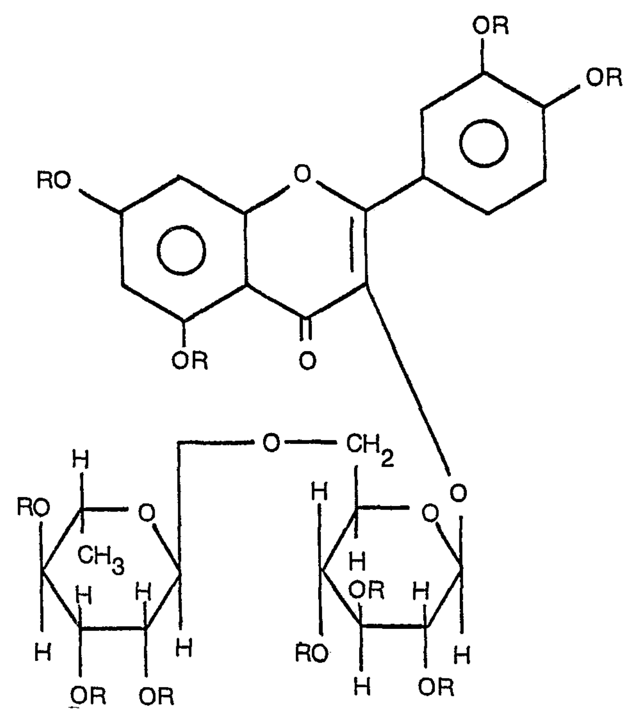

wherein

each R is selected from the group consisting of hydrogen and —$SO_3A$; wherein A is a pharmaceutically acceptable salt cation; with the proviso that at least six of the R groups are —$SO_3A$;

which comprises reacting rutin or a rutin poly(H-)sulfate containing less than six sulfate groups per molecule in a suitable solvent with a trialkylamine-sulfur trioxide complex at 50° to 90°C; isolating the trialkylamine sulfate salt; and if desired converting said trialkylamine sulfate salt into another pharmaceutically acceptable salt.

5. A method according to Claim 4, wherein said suitable solvent is dimethylformamide; said trialkylaminesulfur trioxide complex is trimethylaminesulfur trioxide; and said trialkylamine sulfate salt is converted into an alkali metal or alkaline earth metal salt.

6. A method according to Claim 5, wherein said alkali metal salt is the sodium salt.

7. A pharmaceutical composition suitable for use in inhibiting the complement system in a warm-blooded animal, comprising a rutin poly(H-)sulfate as defined in any one of Claims 1—3 and a pharmaceutically acceptable carrier or diluent.

8. A pharmaceutical composition according to Claim 8, which is formulated as a physically discrete unit suitable for administration as a unitary dosage.

**Claims for the Contracting State: AT**

1. A method for the preparation of a rutin poly(H-)sulfate of the formula:

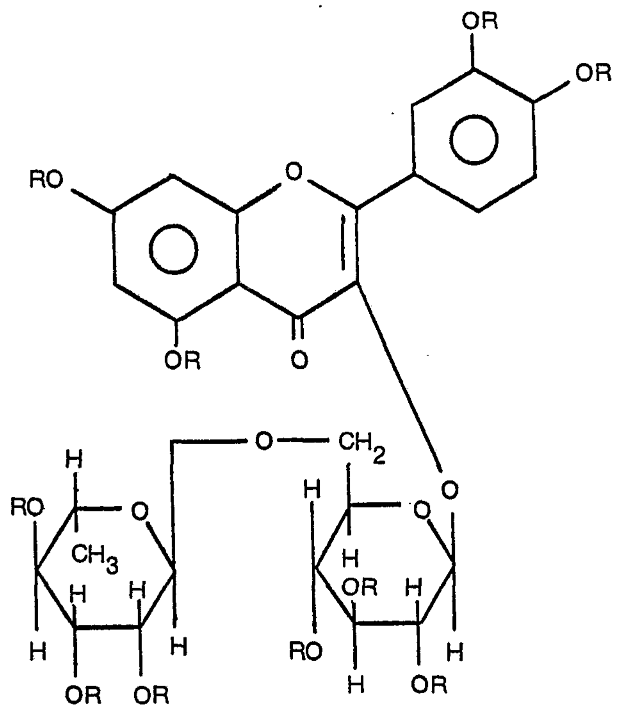

wherein

each R is selected from the group consisting of hydrogen and —$SO_3A$; wherein A is a pharmaceutically acceptable salt cation; with the proviso that at least six of the R groups are —$SO_3A$; which comprises reacting rutin or a rutin poly(H-)sulfate containing less than six sulfate groups per molecule in a suitable solvent with a trialkylamine-sulfur trioxide complex at 50° to 90°C; isolating the trialkylamine sulfate salt; and if desired converting said trialkylamine sulfate salt into another pharmaceutically acceptable salt.

2. A method according to Claim 1, wherein said suitable solvent is dimethylformamide; said trialkylaminesulfur trioxide complex is trimethylaminesulfur trioxide; and said trialkylamine sulfate salt is converted into an alkali metal or alkaline earth metal salt.

3. A method according to Claim 2, wherein said alkali metal salt is the sodium salt.

4. A method of preparing a pharmaceutical composition suitable for use in inhibiting the complement system in a warm-blooded animal, characterized by admixing a pharmaceutically acceptable carrier or diluent with a rutin poly(H-)sulfate of the formula:

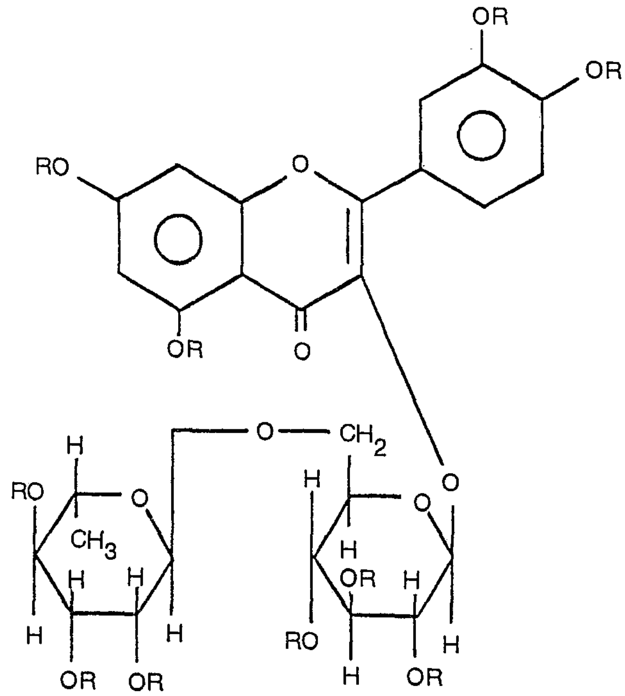

wherein

each R is selected from the group consisting of hydrogen and —$SO_3A$; wherein A is a pharmaceutically acceptable salt cation; with the proviso that at least six of the R groups are —$SO_3A$.

5. A method according to Claim 4, wherein said rutin poly(H-)sulfate is rutin poly(H-)sulfate, trimethylamine salt.

6. A method according to Claim 4, wherein said rutin poly(H-)sulfate is rutin poly(H-)sulfate, sodium salt.

7. A method according to any one of Claims 4—6, wherein the pharmaceutical composition is formulated into a physically discrete unit suitable for administration as a unitary dosage.

**Patentansprüche für die Vertragsstaaten: BE CH DE FR GB IT NL SE**

1. Rutin-poly(H-)sulfat der Formel

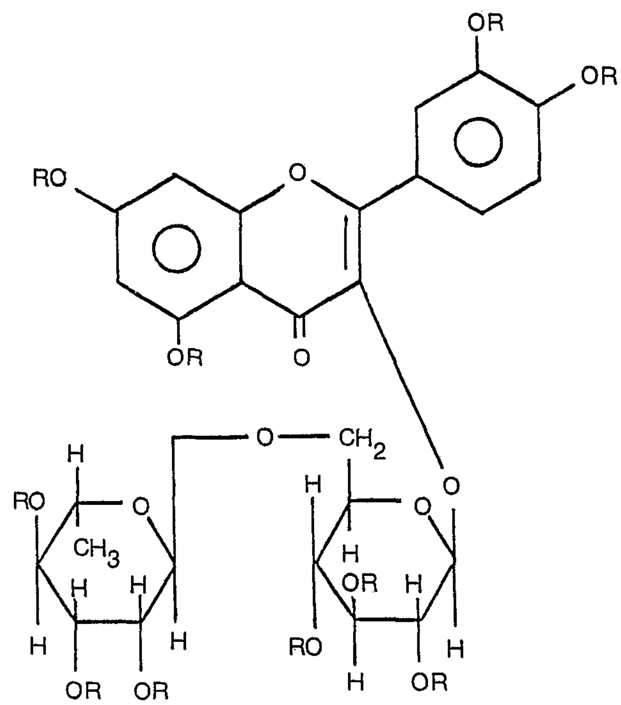

worin jeder Rest R ausgewählt ist aus der Gruppe Wasserstoff und $-SO_3A$; worin A ein pharmazeutisch akzeptables Salzkation bedeutet, unter der Bedingung, daß mindestens sechs der Reste R für $-SO_3A$ stehen.

2. Rutin-poly(H-)sulfat nach Anspruch 1, dadurch gekennzeichnet, daß es sich um Rutin-poly(H-)sulfat-trimethylaminsalz handelt.

3. Rutin-poly(H-)sulfat nach Anspruch 1, dadurch gekennzeichnet, daß es sich um Rutin-poly(H-)sulfat-natriumsalz handelt.

4. Verfahren zur Herstellung eines Rutin-poly(H-)sulfats der Formel

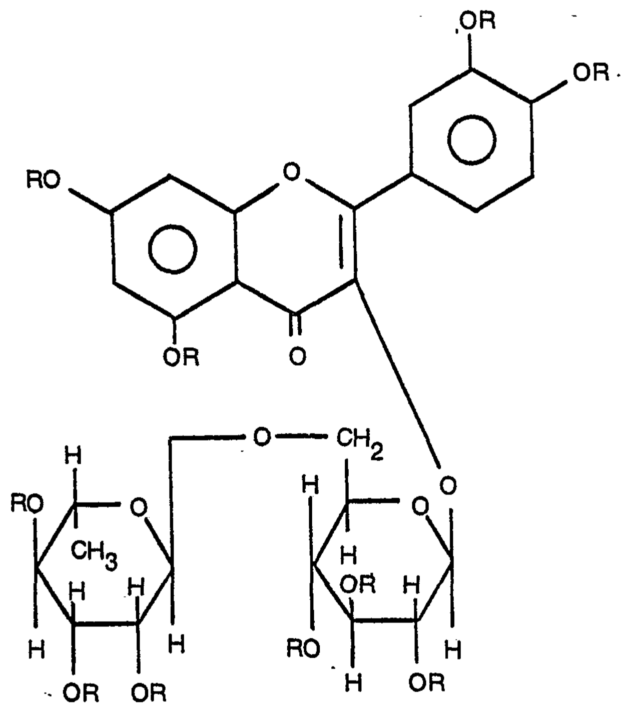

worin jeder Rest R ausgewählt ist aus der Gruppe Wasserstoff und $-SO_3A$; worin A ein pharmazeutisch akzeptables Salzkation bedeutet; unter der Bedingung, daß mindestens sechs der Reste R für $-SO_3A$ stehen, dadurch gekennzeichnet, daß man Rutin oder ein Rutin-poly(H-)sulfat mit weniger als sechs Sulfatgruppen pro Molekül in einem zweckentsprechenden Lösungsmittel mit einem Trialkylamin-Schwefeltrioxid-Komplex bei 50 bis 90°C umsetzt, das Trialkylaminsulfatsalz isoliert und falls erwünscht, das Trialkylaminsulfatsalz in ein anderes pharmazeutisch akzeptables Salz umwandelt.

5. Verfahren nach Anspruch 4, dadurch gekennzeichnet, daß man als zweckentsprechendes Lösungsmittel Dimethylformamid einsetzt und als Trialkylaminschwefeltrioxidkomplex Trimethylschwefeltrioxid und daß man das Trialkylaminsulfatsalz in ein Alkalimetallsalz oder ein Erdalkalimetallsalz umwandelt.

6. Verfahren nach Anspruch 5, dadurch gekennzeichnet, daß das Alkalimetallsalz ein Natriumsalz ist.

7. Pharmazeutisches Mittel zur Inhibierung des Komplementsystems in Warmblüter, gekennzeichnet durch einen Gehalt an einem Rutin-poly(H-)sulfat nach einem der Ansprüche 1 bis 3 und einem pharmazeutisch akzeptablen Trägerstoff oder Verdünnungsmittel.

8. Pharmazeutisches Mittel nach Anspruch 7, dadurch gekennzeichnet, daß es in Form diskreter Einheiten für die Verabreichung als Dosiseinheit vorliegt.

**Patentansprüche für den Vertragsstaat: AT**

1. Verfahren zur Herstellung eines Rutin-poly(H-)sulfats der Formel

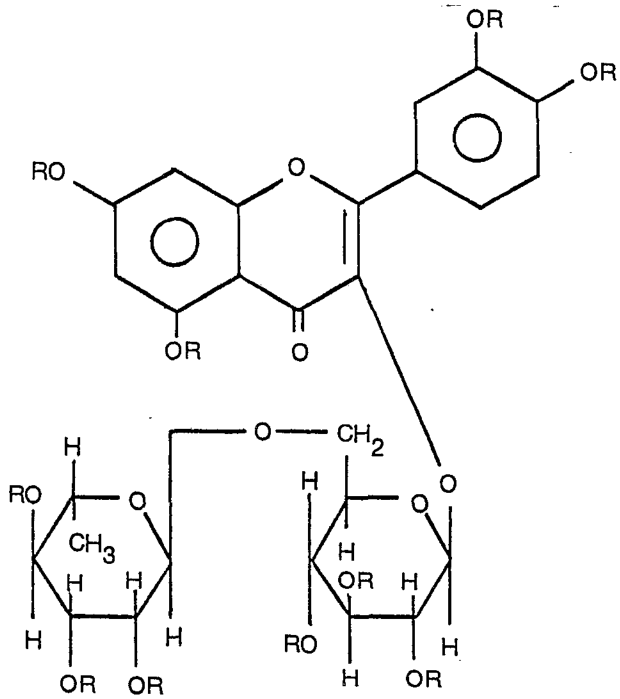

worin jeder Rest R ausgewählt ist aus der Gruppe Wasserstoff und $—SO_3A$; worin A ein pharmazeutisch akzeptables Salzkation bedeutet; unter der Bedingung, daß mindestens sechs der Reste R für $—SO_3A$ stehen, dadurch gekennzeichnet, daß man Rutin oder ein Rutin-poly(H-)sulfat mit weniger als sechs Sulfatgruppen pro Molekül in einem zweckentsprechenden Lösungsmittel mit einem Trialkylamin-Schwefeltrioxid-Komplex bei 50 bis 90°C umsetzt, das Trialkylaminsulfatsalz isoliert und falls erwünscht, das Trialkylaminsulfatsalz in ein anderes pharmazeutisch akzeptables Salz umwandelt.

2. Verfahren nach Anspruch 1, dadurch gekennzeichnet, daß man als zweckentsprechendes Lösungsmittel Dimethylformamid einsetzt und als Trialkylaminschwefeltrioxidkomplex Trimethylschwefeltrioxid und daß man das Trialkylaminsulfatsalz in ein Alkalimetallsalz oder ein Erdalkalimetallsalz umwandelt.

3. Verfahren nach Anspruch 2, dadurch gekennzeichnet, daß das Alkalimetallsalz ein Natriumsalz ist.

4. Verfahren zur Herstellung eines pharmazeutischen Mittels zur Inhibierung des Komplementsystems in Warmblütern, gekennzeichnet durch Vermischung eines pharmazeutisch akzeptablen Trägers oder Verdünnungsmittels mit einem Rutin-poly(H-)sulfat der Formel

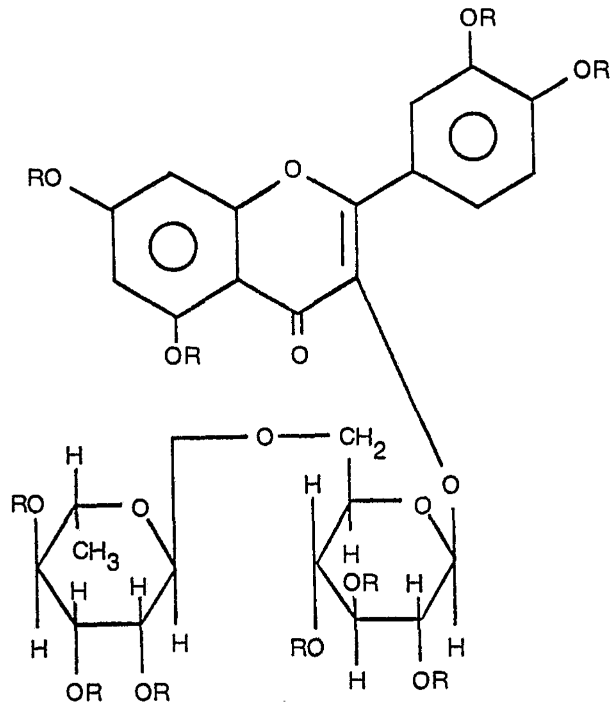

worin jeder Rest R ausgewählt ist aus der Gruppe Wasserstoff und —$SO_3A$; wobei A ein pharmazeutisch akzeptables Salzkation bedeutet, unter der Bedingung, daß mindestens sechs der Reste R für —$SO_3A$ stehen.

5. Verfahren nach Anspruch 4, dadurch gekennzeichnet, daß man als Rutin-poly(H-)sulfat das Trimethylaminsalz des Rutin-poly(H-)sulfats einsetzt.

6. Verfahren nach Anspruch 4, dadurch gekennzeichnet, daß man als Rutin-poly(H-)sulfat das Natriumsulfat des Rutin-poly(H-)sulfats einsetzt.

7. Verfahren nach einem der Ansprüche 4 bis 6, dadurch gekennzeichnet, daß das pharmazeutische Mittel in einer diskreten Einheit zur Verabreichung als Einheitsdosis vorliegt.

**Revendications pour les Etats contractants: BE CH DE FR GB IT NL SE**

1. Poly (H-) sulfate de rutine de formule

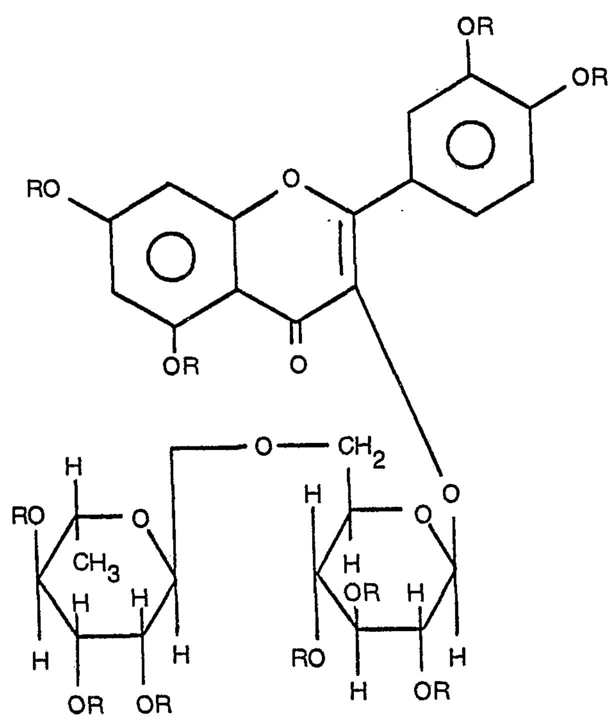

dans lequel chaque R est choisi parmi l'hydrogène et —$SO_3A$; A étant un cation d'un sel pharmaceutiquement acceptable avec la condition qu'au moins six des groupes R sont —$SO_3A$.

2. Poly (H-) sulfate de rutine selon la revendication 1, qui est le sel de triméthylamine du poly (H-) sulfate de rutine.

3. Poly (H-) sulfate de rutine selon la revendication 1, qui est le sel de sodium du poly (H-) sulfate de rutine.

4. Procédé pour la préparation d'un poly (H-) sulfate de rutine de formule

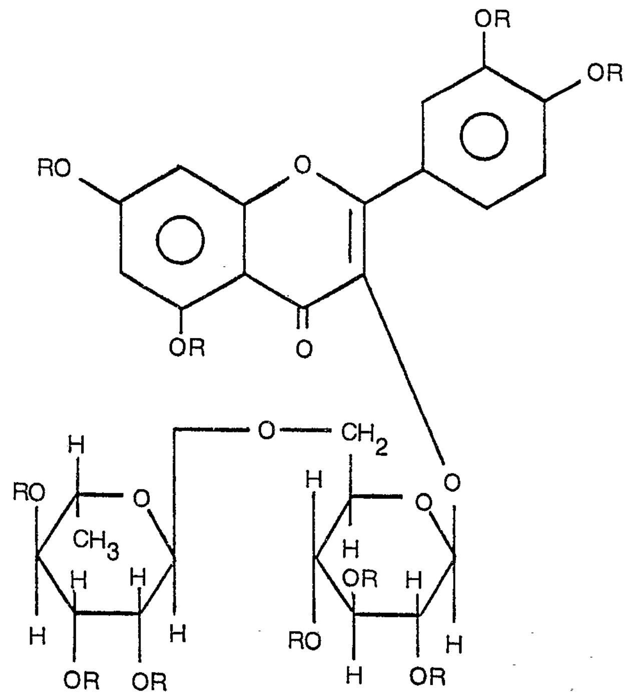

dans lequel chaque R est choisi parmi l'hydrogène et —$SO_3A$, A étant un cation d'un sel pharmaceutiquement acceptable avec la condition qu'au moins six des groupes R sont —$SO_3A$; ce procédé comprenant la réaction de la rutine ou d'un poly (H-) sulfate de rutine contenant moins de six groupes sulfate par molécule dans un solvant convenable avec un complexe trialkylaminetrioxyde de soufre à 50° à 90°C; l'isolement du sel de trialkylamine du sulfate; et si on le souhaite, la conversion dudit sel de trialkylamine dudit sulfate en un autre sel pharmaceutiquement acceptable.

5. Procédé selon la revendication 4 dans lequel ledit solvant convenable est le diméthylformamide; ledit complexe alkylamine-trioxyde de soufre est le triméthylaminetrioxyde de soufre; et ledit sel de trialkylamine dudit sulfate est transformé en un sel d'un métal alcalin ou d'un métal alcalino-terreux.

6. Procédé selon la revendication 5, dans lequel ledit sel de métal alcalin est le sel de sodium.

7. Composition pharmaceutique convenant pour être utilisée dans l'inhibition du système du complément chez un animal à sang chaud, comprenant un poly (H-) sulfate de rutine tel que défini dans l'une quelconque des revendications 1 à 3 et un véhicule ou diluant pharmaceutiquement acceptable.

8. Composition pharmaceutique selon la revendication 8, qui est formulée sous forme d'une unité séparée du point de vue physique convenant pour l'administration comme dose unitaire.

**Revendications pour l'Etat contractant: AT**

1. Procédé pour la préparation d'un poly (H-) sulfate de rutine de formule

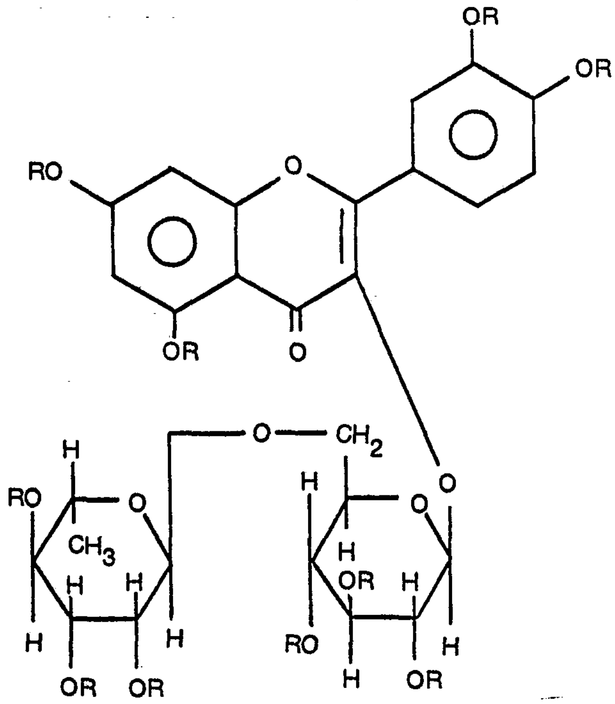

dans lequel chaque R est choisi parmi l'hydrogène et —$SO_3A$, A étant un cation d'un sel pharmaceutiquement acceptable, avec la condition qu'au moins six des groupes R sont —$SO_3A$; ce procédé comprenant la réaction de la rutine ou d'un poly (H-) sulfate de rutine contenant moins de six groupes sulfate par molécule dans un solvant convenable avec un complexe trialkylaminetrioxyde de soufre à 50° à 90°C; l'isolement du sel de trialkylamine du sulfate; et si on le souhaite, la conversion dudit sel de trialkylamine dudit sulfate en un autre sel pharmaceutiquement acceptable.

2. Procédé selon la revendication 1 dans lequel ledit solvant convenable est le diméthylformamide; ledit complexe alkylamine-trioxyde de soufre est le triméthylaminetrioxyde de soufre; et ledit sel de trialkylamine dudit sulfate est transformé en un sel d'un métal alcalin ou d'un métal alcalino-terreux.

3. Procédé selon la revendication 2, dans lequel ledit sel de métal alcalin est le sel de sodium.

4. Procédé de préparation d'une composition pharmaceutique convenant, pour être utilisée dans l'inhibition du système du complément chez un animal à sang chaud, caractérisé en ce que l'on mélange un véhicule ou diluant pharmaceutiquement acceptable avec un poly (H-) sulfate de rutine de formule

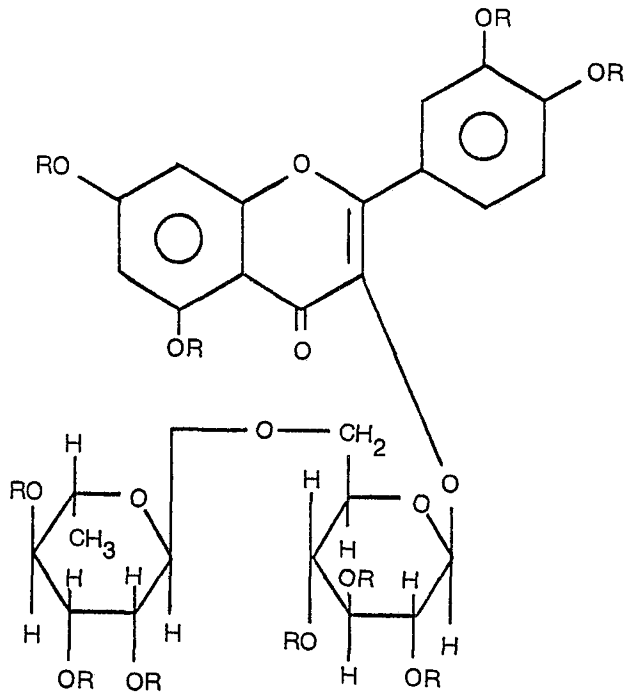

dans lequel chaque R est choisi parmi l'hydrogène et $-SO_3A$; A étant un cation d'un sel pharmaceutiquement acceptable avec la condition qu'au moins six des groupes R sont $-SO_3A$.

5. Procédé selon la revendication 4, dans lequel le poly (H-) sulfate de rutine est le sel de triméthylamine de poly (H-) sulfate de rutine.

6. Procédé selon la revendication 4, dans lequel de poly (H-) sulfate de rutine est le sel de sodium de poly (H-) sulfate de rutine.

7. Procédé selon l'une quelconque des revendications 4 à 6, dans lequel la composition pharmaceutique est formulée sous forme d'unités séparées du point de vue physique convenant pour l'administration comme dose unitaire.